# EUROPEAN PATENT APPLICATION

(11) **EP 1 992 616 A1**
(43) Date of publication of application: **19.11.2008**
(21) Application number: 08015098.0
(22) Date of filing: 14.07.2006
(51) Int. Cl.: C07D 211/32

(54) **Process for the preparation of donepezil and intermediate compounds thereof as well as hydrates of donepezil**

(30) Priority: 30.07.2005 GB 0515803
(62) Divisional of application: 06764973.1
(71) Applicant: Pliva Hrvastka D.O.O., 10000 Zagreb (HR)
(72) Inventor: Manikowski, Andrzej, 31546 Krakow (PL); Ziobro, Barbara, 31546 Krakow (PL); Zaba, Andrzej, 31546 Krakow (PL); Makosza, Myeczislaw, 01496 Warszawa (PL); Jerkovic, Juraj, 10000 Zagred (CR); Grebenar, Ivica, 10000 Zagred (CR); Mestrovic, Ernest, 10000 Zagred (CR); Samardzic, Zrinka Mastelic, 10000 Zagred (CR); Lerman, Lidija, 10000 Zagred (CR); Kaczorowska, Katarzyna, 31546 Krakow (PL)
(74) Representative: Bucks, Teresa Anne

(57) **Abstract**

The present invention relates to a new process for the preparation of intermediate compounds useful in the manufacture of donepezil, or a pharmaceutically-acceptable salt thereof, and also relates to processes for preparing donepezil and to the novel intermediates per se. In addition the invention relates to various hydrated forms of donepezil.

## Description

The present invention relates to a new process for the preparation of donepezil, or a pharmaceutically-acceptable salt thereof, and processes for the preparation of intermediate compounds useful in the manufacture of donepezil, or a pharmaceutically-acceptable salt thereof, and also relates to novel intermediates per se.

Donepezil (1-benzyl-4-(5,6-dimethoxyindanone-2-yl)methylpiperidine) is a pharmaceutically active compound. Its structure is shown in Formula I.

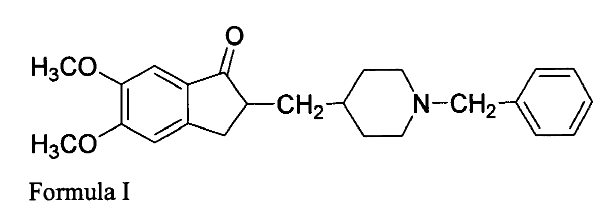

Donepezil belongs to a group of selective acetylcholinesterase inhibitors used for the treatment of mild to moderate Alzheimer's disease. Symptoms of the disease include memory loss, confusion, personality changes, disorientation and loss of language skills.

Several methods for the preparation of intermediates useful in donepezil synthesis have been described.

International Publication No. WO 97/22584 and U.S. Patent No. 6,649,765 describes a process for the preparation of donepezil from 4-[5,6-dimethoxy-1-indanon)-2-yl]methylpiperidine (Formula II).

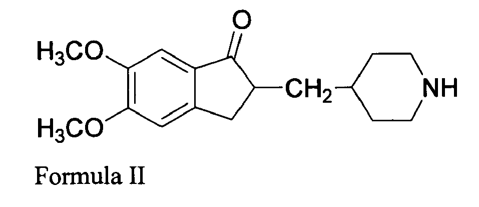

U.S. Patent No. 5,606,064 and European Publication No. EP 711,756 describes a process for the preparation of donepezil from pyridinium salt having Formula III.

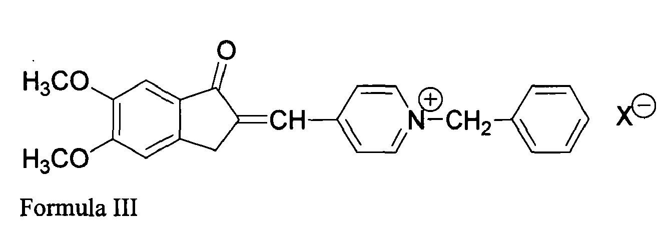

International Publication No. WO 99/36405 and US Patent No. 6,252,081 describes the preparation of donepezil from quaternary salt having Formula IV.

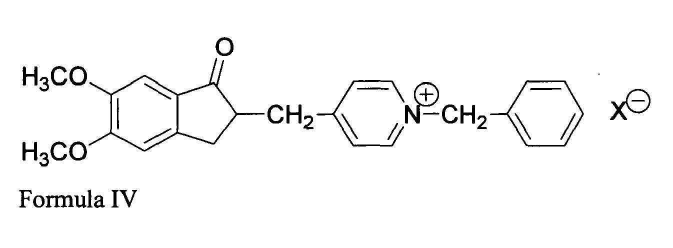

European Publication No. EP 296,560, U.S. Patent No. 4,895,841 and U.S. Patent No. 5,100,901 reports the preparation of donepezil from 1-benzyl-4-[(5,6-dimethoxy-1-indanon)-2-ylidenyl-methylpiperidine (Formula V).

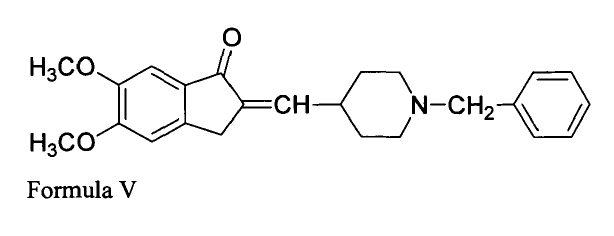

European Publication No. EP 1,386,607 provides a process for the preparation of donepezil from a compound of the Formula VI.

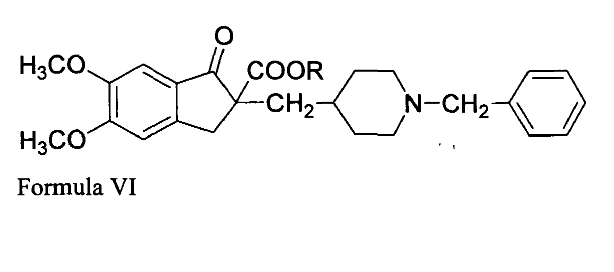

International Publication No. WO 00/09483 and U.S. Patent No. 6,492,522 present a process for the preparation of donepezil from compounds of the Formula VII and Formula VIII.

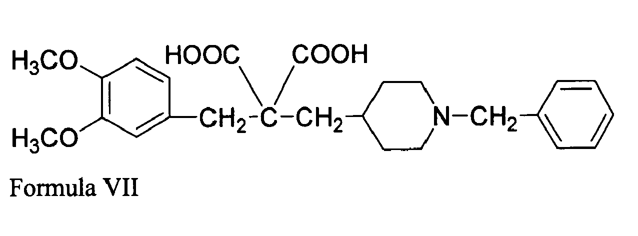

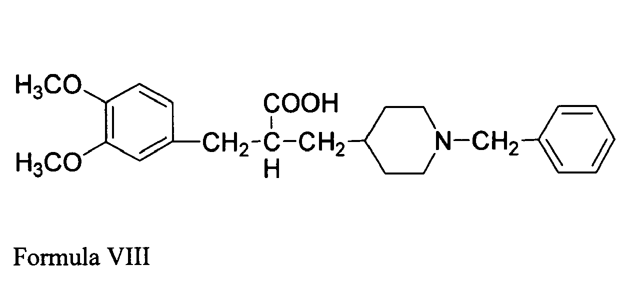

We have found that the compound of Formula IX is an excellent intermediate in the production of donepezil, or a pharmaceutically-acceptable salt thereof. 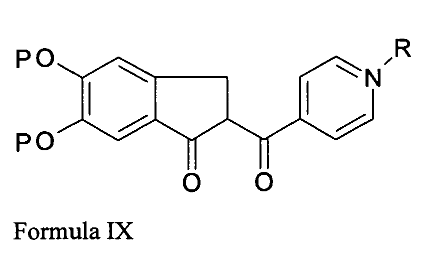 wherein P is a precursor group capable of conversion to a methyl group, alternatively P is a methyl group, and R is a precursor group capable of conversion to a benzyl group or R is a hydrogen atom, alkyl [preferably (C1-4)alkyl] or an aralkyl [preferably, benz(C1-4)alkyl].

It will be apparent to the skilled person viewing the structure of the intermediate of Formula IX that the two carbonyls, the first carbonyl being on the indanone and the second carbonyl being at the bridge between the indanone and pyridine ring, should not allow such a compound to be a suitable intermediate for producing donepezil, since *prima facie* it would appear unlikely that the controlled reduction of the second carbonyl group, at least in a significant yield, could occur without also reducing the first carbonyl group at the same time.

We have surprisingly found that the second carbonyl group can be highly selectively reduced without any significant reduction in the first carbonyl group. The advantages of this process, over the known processes for making donepezil, is that it uses widely available convenient, stable and cheaper starting materials and reagents, enabling considerably lower production costs than previously published processes

The invention relates to a process for the preparation of donepezil, or a pharmaceutically-acceptable salt thereof, which process comprises;
optionally converting a compound of Formula IX with another compound to form a compound of Formula XII (wherein R is not the same as T) 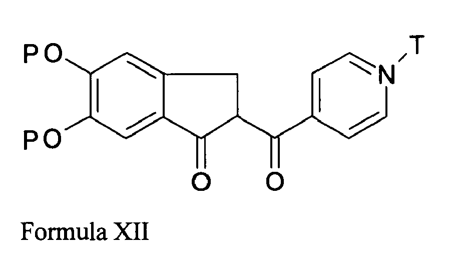 P is a precursor group capable of conversion to a methyl group, alternatively P is a methyl group, wherein T is a precursor group capable of conversion to a benzyl group or T is a hydrogen atom, alkyl [preferably (C1-4)alkyl] or an aralkyl [preferably benz(C1-4)alkyl], preferably T is a benzyl group;
reducing the compound of Formula XII to obtain compound of Formula XIII 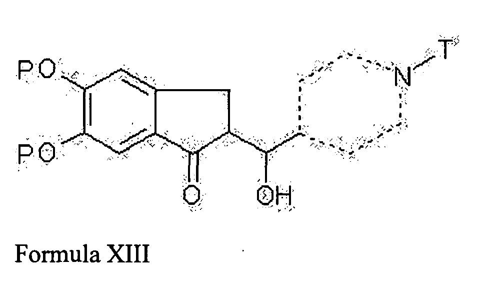 wherein the dotted lines represent a bond that may exist as double or as a single bond [preferably only one bond is a double bond in the ring, alternatively all of the bonds in the ring are saturated], wherein P is a precursor group capable of conversion to a methyl group, alternatively P is a methyl group and wherein T is a precursor group capable of conversion to a benzyl group or T is a hydrogen atom, alkyl [preferably (C1-4)alkyl] or an aralkyl [preferably, benz(C1-4)alkyl], preferably T is a benzyl group;
dehydrating and subsequently reducing a compound of Formula XIII where P is a methyl group and T is a benzyl group to obtain a compound of Formula I (donepezil, or a pharmaceutically-acceptable salt thereof), or additionally
where P is methyl group and T is a precursor group capable of conversion to a benzyl group , converting the T to benzyl group to obtain donepezil, or a pharmaceutically-acceptable salt thereof, or
where P is a precursor group capable of conversion to a methyl group and T is a benzyl group, then converting P to a methyl group to obtain donepezil, or a pharmaceutically-acceptable salt thereof, or
where P is a precursor group capable of conversion to a methyl group and T is a precursor group capable of conversion to a benzyl group , undertaking the above described steps of converting P to a methyl group and converting group T to a benzyl group in either order, to obtain donepezil, or a pharmaceutically-acceptable salt thereof.

The process also comprises;
direct reduction of the compound of Formula XII where P is methyl group and T is a benzyl group to obtain a compound of Formula I (donepezil, or a pharmaceutically-acceptable salt thereof) without isolating Formula XIII as an intermediate; or additionally
where P is methyl group and T is a precursor group capable of conversion to a benzyl group, converting the group T to benzyl group to obtain donepezil, or a pharmaceutically-acceptable salt thereof, or
where P is a precursor group capable of conversion to a methyl group and T is a benzyl group, then converting P to a methyl group to obtain donepezil, or a pharmaceutically-acceptable salt thereof, or
where P is a precursor group capable of conversion to a methyl group and T is a precursor group capable of conversion to a benzyl group, undertaking the above described steps of converting P to a methyl group and converting group T to benzyl group in either order, to obtain donepezil, or a pharmaceutically-acceptable salt thereof.

Conversion of the group T to a benzyl group, when necessary, involves the use of standard chemical techniques within the understanding of the skilled person.

Conversion of the precursor group P to a methyl group, when necessary, involves the use of standard chemical techniques within the understanding of the skilled person.

The invention relates to process for the preparation of donepezil utilising as an intermediate in the process a compound of Formula IX 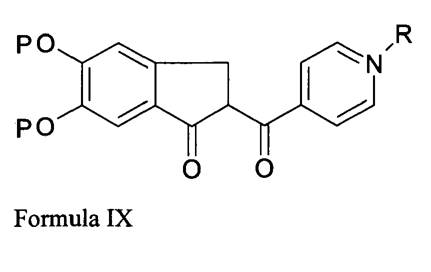 wherein P is a precursor group capable of conversion to a methyl group, alternatively P is a methyl group, and R is a precursor group capable of conversion to a benzyl group or R is a is a hydrogen atom, alkyl [preferably (C1-4)alkyl] or an aralkyl [preferably benz(C1-4)alkyl], in either of its tautomeric forms or in the form of any salt thereof.

The invention relates to a compound of Formula IX, as defined above.

The present invention relates to a process for preparing novel intermediates of Formula IX (useful in the synthesis of pharmaceutical compounds, including donepezil, or a pharmaceutically-acceptable salt thereof), the process comprising reacting a compound of Formula X 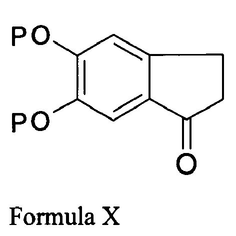 wherein P is as defined above with a compound of Formula XI 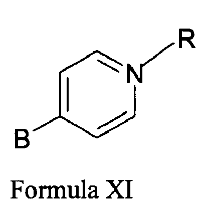 wherein R is as defined above within a compound of Formula IX

Preferably the process is conducted;
in the presence of alkali metal alkoxide, such as sodium methoxide, or an alkali metal hydride, such as sodium hydride;
in the presence of aliphatic alcohols as an initiator;
at an elevated temperature in the range of from 100°C to 180°C, ideally at reflux; in the presence of solvent or without solvent, preferably in an aromatic solvent.

Preferred solvents are selected from cyclohexanol, butanol, toluene or a mixture thereof.

The invention relates to a process for the preparation of donepezil, or a pharmaceutically-acceptable salt thereof, utilising as an intermediate in the process a compound of Formula XIII 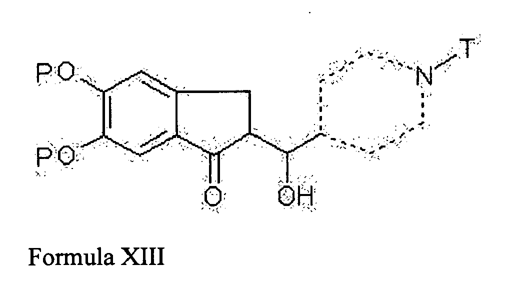 wherein the dotted lines represent a bond that may exist as double or as a single bond [preferably only one bond is a double bond in the ring alternatively all of the bonds are saturated], wherein P is a precursor group capable of conversion to a methyl group, alternatively P is a methyl group and wherein T is a precursor group capable of conversion to a benzyl group or T is a hydrogen atom, alkyl [preferably (C1-4)alkyl] or an aralkyl [preferably benz(C1-4)alkyl], preferably T is a benzyl group, in either of its tautomeric forms or in the form of any salt thereof.

Optionally, the carbonyl group on indanone in the compounds of Formula IX, XII and XIII is protected.

The invention further relates to a compound of Formula XIII, as defined above.

It is clear to those skilled in the art that the compounds of Formula IX, XII, XIII, may exist in their alternative tautomeric form as well as in the form of a salt, preferably a halogenide salt.

The starting materials, 5,6-dimethoxy-1-indanone and isonicotinic acid derivatives are generally available chemicals applied for a number of purposes in the chemical industry.

As used herein the term alkyl preferably means alkyl (C1-4)alkyl (individually selected from methyl, ethyl, propyl, butyl).

As used herein the term aralkyl preferably means benz(C1-4)alkyl (individually selected from benzyl, benzethyl, benzpropyl and benzbutyl.

P is preferably a methyl group. Preferably both P groups are methyl groups.

A preferred pharmaceutically acceptable salt is the hydrochloride salt.

The process is described in Scheme 1

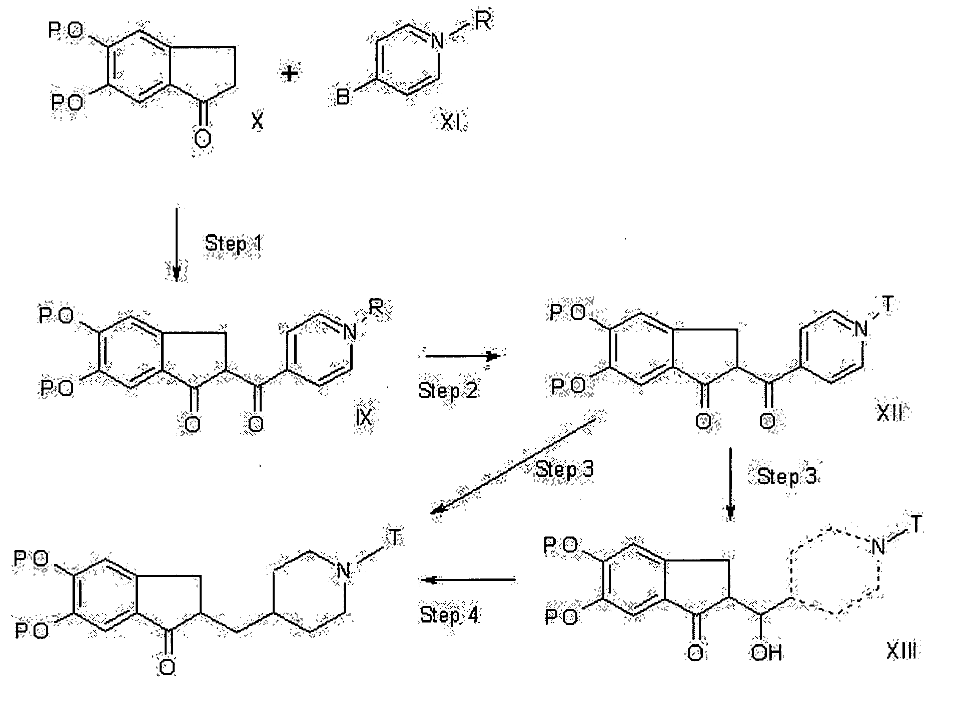

Preferably step 1) of Scheme 1 is conducted by performing a mixed Claisen condensation reaction consisting of reacting 5,6-dimethoxy-1-indanone and isonicotinate ester in the presence of alkali metal alkoxides, preferable sodium methoxide or alkali metal hydrides, preferable sodium hydride, and in the presence of heat, preferably at reflux. Suitable solvents include those described in the examples and are preferably aromatic solvents, such as toluene. These solvents may be used alone or as a mixture thereof.

Preferably step 2) of Scheme 2 is conducted by performing benzylation with a benzyl halide.

Preferably either step 3) of Scheme 1 is conducted by performing reduction with a hydride or by hydrogenation in the presence of a noble metal or its oxide as a catalyst.

Preferably step 4) of Scheme 1 is performed by adding an acid and heating followed by reduction with hydrogen in the presence of a noble metal or its oxide as a catalyst or by reduction with a reducing agent e.g. ammonium formate.

Reduction reactions, such as step 3 in Schemes 1 and 2 are conducted in the presence of a hydride, preferably sodium borohydride or by employment of hydrogen or a source of hydrogen with the presence of a metal catalyst, preferably, Pt, Pd, Ni or their compounds and alloys

Hydrogenation reactions, such as step 4 in Schemes 1 and 2, are conducted with employment of hydrogen or a source of hydrogen. Preferably the hydrogenation is conducted with the presence of a metal catalyst, preferably, Pt, Pd, Ni or their compounds and alloys

Preferably, donepezil is obtained by Scheme 2:

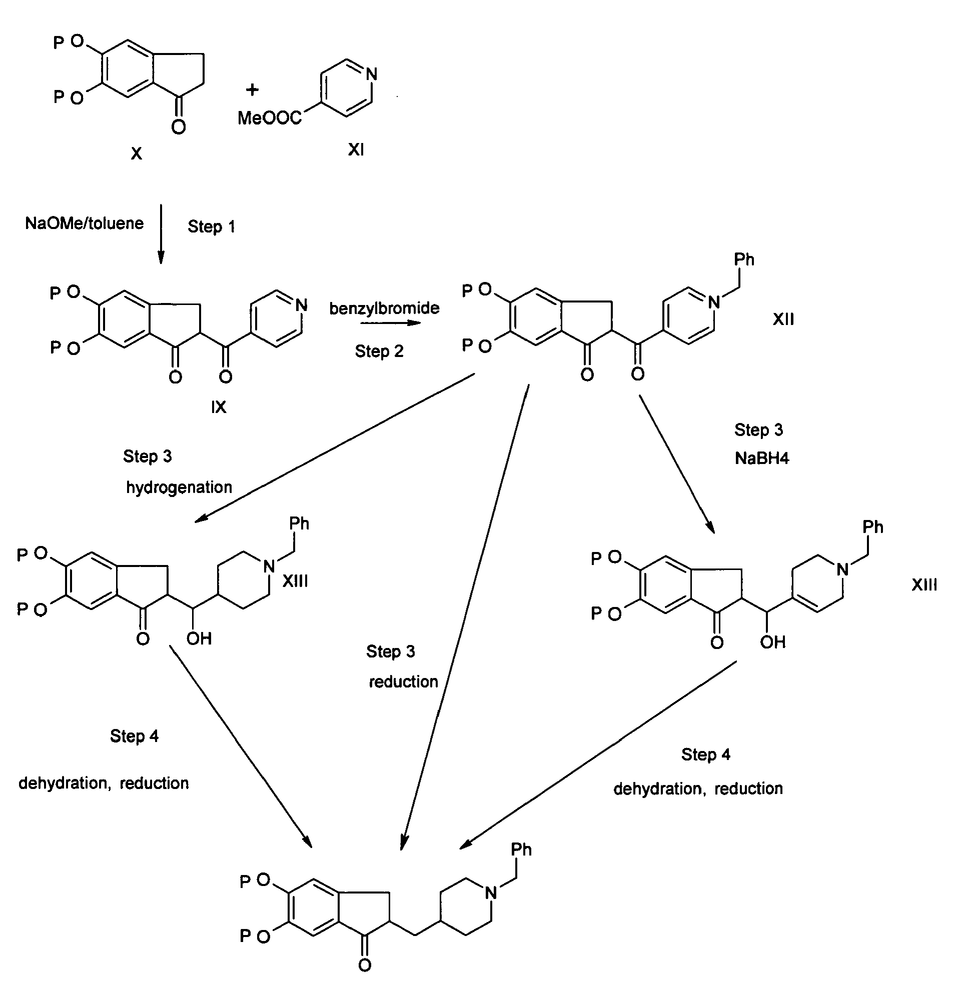

We present as a feature of the invention a number of different hydrates of donepezil. These hydrates may be prepared from the donepezil produced by the present invention, or may be produced from donepezil from any other route of synthesis.

Donepezil hydrochloride hemihydrate. Preferably the donepezil hydrochloride hemihydrate has a water content of from 1.5% weight to 2.8 % weight, ideally 2.0% weight.

Donepezil hydrochloride monohydrate. Preferably the donepezil hydrochloride monohydrate has a water content of from 3.5 % weight to 4.6 % weight, ideally 4.1 % weight.

Donepezil hydrochloride sesquihydrate. Preferably the donepezil hydrochloride sesquihydrate has a water content of from 5.5% weight to 6.5 % weight, ideally 6.1 % weight.

Donepezil hydrochloride dihydrate. Preferably the donepezil hydrochloride dihydrate has a water content of from 7.5 % weight to 8.5 % weight, ideally 8.2% weight.

As stated above all water contents denote water of crystallisation.

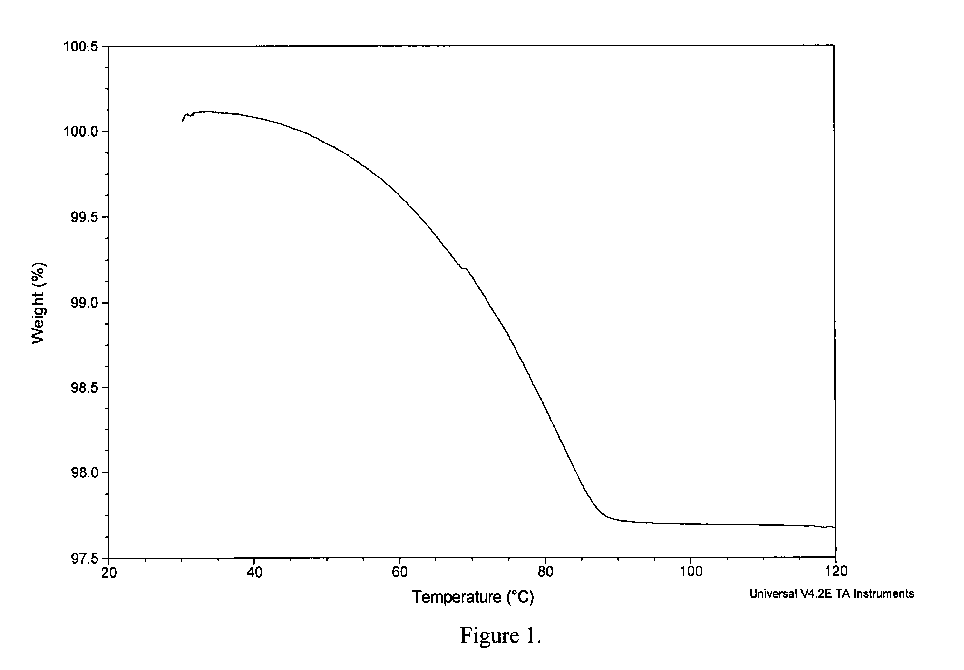

- Figure 1.: shows a typical thermo-gravimetric analysis [TGA] thermogram for donepezil hydrochloride hemihydrate.

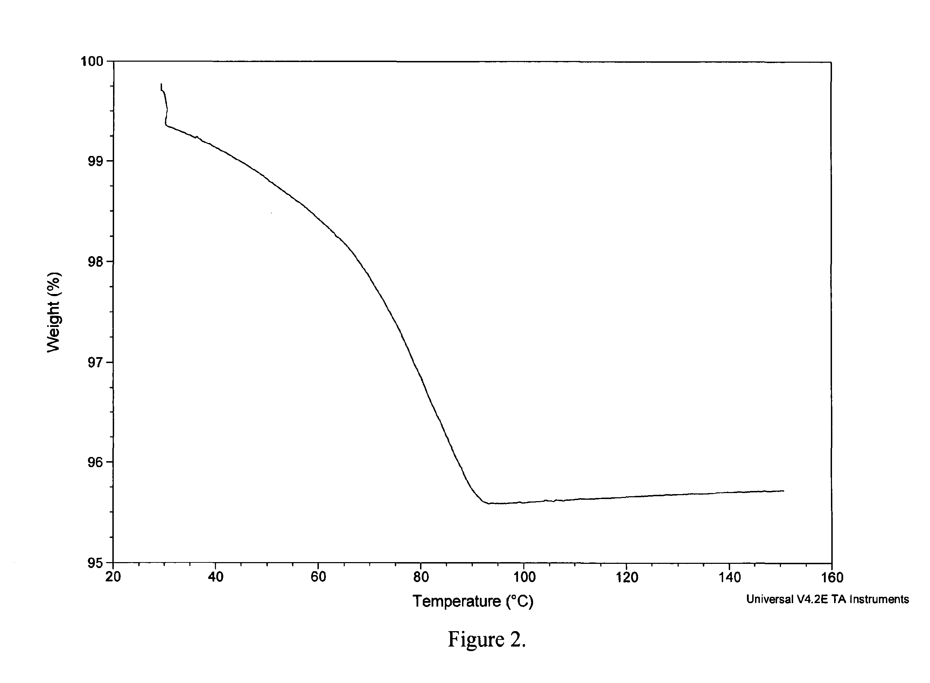

- Figure 2.: shows a typical TGA thermogram for donepezil hydrochloride monohydrate.

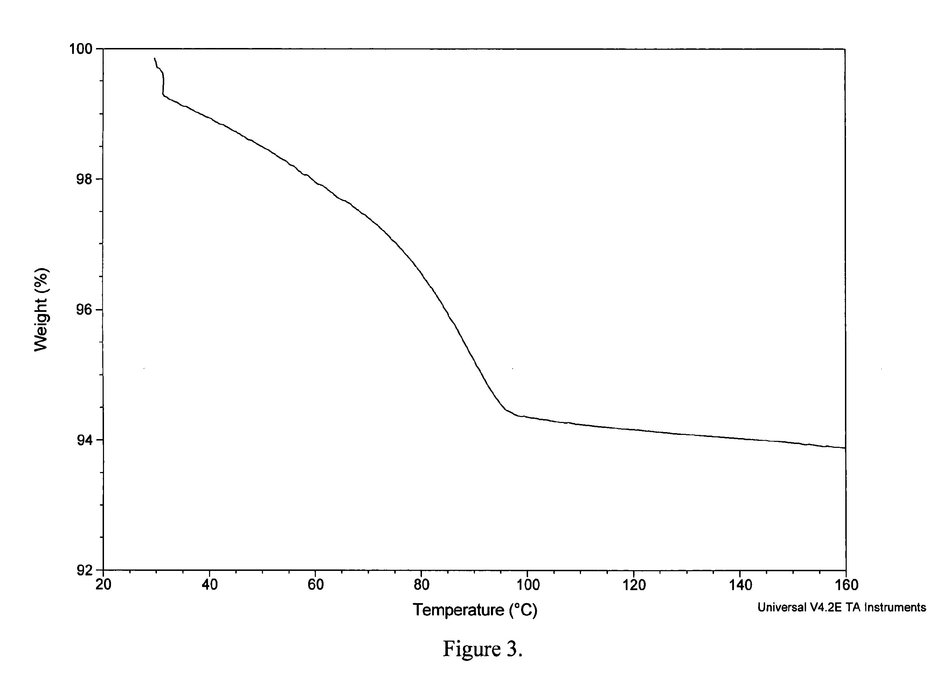

- Figure 3.: shows a typical TGA thermogram for donepezil hydrochloride sesquihydrate.

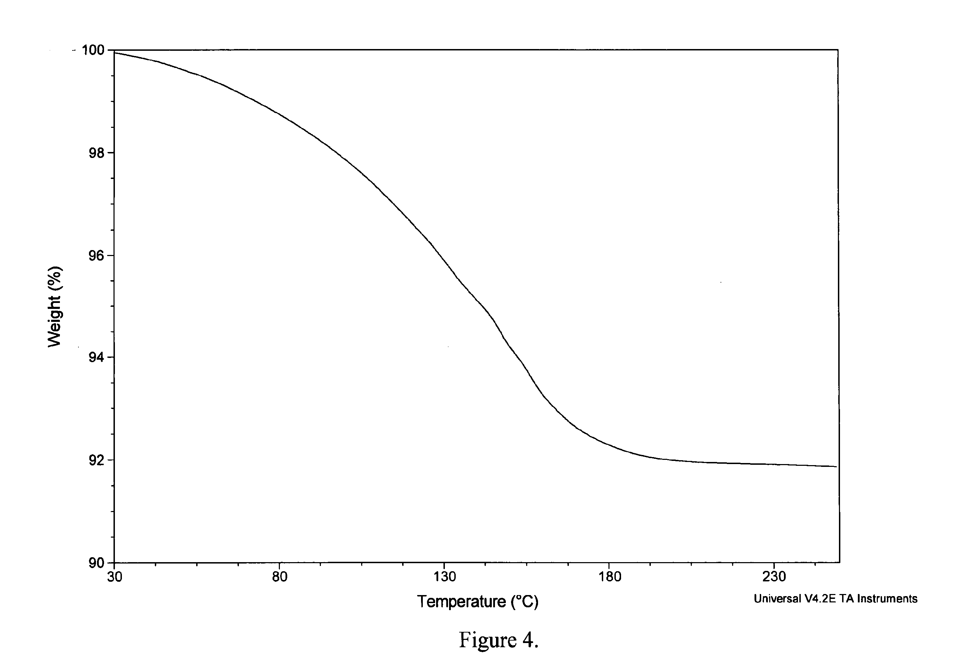

- Figure 4.: shows a typical TGA thermogram for donepezil hydrochloride dihydrate.

Different physical forms of a drug substance can have different chemical and physical properties, including melting point, chemical reactivity, apparent solubility, dissolution rate, optical and mechanical properties, vapour pressure, and density. These properties can have a direct effect on the ability to process and/or manufacture a drug substance and a drug product, as well as on drug product stability, dissolution, and bioavailability. Thus, the physical form of the drug can affect the quality, safety, and efficacy of a drug product.

Different physical forms can include crystalline and amorphous forms as well as solvate and hydrate forms, which can be further characterised as follows.

There are a number of methods that can be used to characterise different physical of a drug substance. In relation to characterising the hydration state of a drug, such as donepezil, it is preferred to use thermal analysis (e.g., differential scanning calorimetry, thermal gravimetric analysis [TGA], and hot-stage microscopy) so that the water content in the crystal structure can be directly measured. Spectroscopy (e.g., infrared [IR], Raman, solid-state nuclear magnetic resonance [ssNMR]) as well as other techniques can be helpful to further characterise the physical forms. Single crystal X-ray diffraction is currently regarded as the definitive evidence of polymorphism. However, it is possible that drugs having different states of hydration may have similar or identical single crystal structures and therefore thermal analysis provides the most accurate identification of the type of hydrated from, although X-ray analysis can provide helpful additional information about the hydrated form. X-ray powder diffraction can also be used to support the existence of polymorphs.

Physical forms, even slightly different states of hydration of a drug substance, can exhibit different chemical, physical and mechanical properties as referred to above, including aqueous solubility and dissolution rate, hygroscopic, particle shape, density, flowability, and compactibility, which in turn may affect processing of the drug substance and/or manufacturing of the drug product. Different hydrates can also exhibit different stabilities. The most stable physical form of a drug substance is often chosen during drug development based on the minimal potential for conversion to another form and on its greater chemical stability and reduces variability of physical properties during storage. However, a meta-stable form can alternatively be chosen for various reasons, including bioavailability enhancement, provided that the means for stabilising the meta-stable form can be found.

In the present application the different hydrate forms of donepezil have particular advantages in that they have good solubility and dissolution properties, in addition to those properties described above.

The invention is illustrated by the following examples

The present invention is not to be limited in scope by the specific embodiments described herein. Various modifications of the invention in addition to those described herein will become apparent to those skilled in the art from the foregoing description. Such modifications are intended to fall within the scope of the appended claims.

### Example 1 - Preparation of 5,6-dimethoxy-2-(pyridine-4-carbonyl)indan-1-one (Formula IX)

5,6-Dimetoxyindanone (0.5g), isonicotinic acid butyl ester (0.56g) and toluene (5ml) were mixed together and treated with sodium hydride (0.1g). After being stirred for 10 minutes at room temperature, heating was applied and the temperature was raised up to a gentle reflux. The reaction was carried out for 0.5h and cooled down to room temperature. 5% HCl aqua solution was added (3ml), all was stirred for 5min and pH was next brought to neutral with saturated sodium bicarbonate. After separation of the toluene fraction, the water layer was washed with chloroform (3x15ml) and chloroform organic layers were dried over anhydrous sodium sulfate. Further removing of solvent under vacuum gave crude product as tiny yellow-orange crystals. Maceration in hot toluene gave 0.4g of product (52%).
EI-MS: 297; ¹H NMR (CDCl₃,200 MHz) 8.77 (d, 2), 7.75 (dd, 2), 7.32 (s, 1), 6.99 (s, 1), 3.99 (s, 3), 3.96 (s, 3), 3.87 (s, 2); mp. 203°-206°C.

### Example 2 - Preparation of 5,6-dimethoxy-2-(pyridine-4-carbonyl)indan-1-one (Formula IX)

5,6-Dimetoxyindanone (50.0g), isonicotinic acid methyl ester (41.0g), toluene (600cm³) and methanol (10cm³) were added into reactor equipped with thermometer and mechanical stirrer. After being stirred for 10 minutes at room temperature sodium methoxide (16.9g) was added in one portion. Heating mantel was applied and the temperature was raised up till reflux (84°÷85°C). At this point methanol was being gradually distilled out, so temperature was slowly raised within 2 hours until the temperature reached 101°-103°C. At this point the reaction was continued for 0.5 hour under reflux and next cooled down to room temperature using a cold-water bath. To the obtained suspension was added water (500 cm³) and after being well mixed for 5 minutes, a dark brown 2-phase solution was separated on a separator funnel. Toluene layer was returned to reactor and mixed with 1% NaOH aqua solution (200cm³). After further separation, both water layers were combined together and treated with 10% hydrochloric acid aqua solution to bring pH to 4.0-4.5 followed by addition of water (500cm³). A dense yellow solid of product was next filtered off by suction on Büchner funnel, washed with water (500cm³) and methanol (350cm³). After drying, yellow amorphous solid was treated with toluene (300cm³), stirred at reflux for 60 minutes, cooled down to 20°C and stirred for next 1 hour. The tiny orange-yellow crystals of product were filtered off and washed with toluene (150cm³). After being dried for 4 hours at 65-70°C under reduced pressure (0.04MPa), 63g of product was obtained (82%).
EI-MS: 297; ¹H NMR (CDCl₃, 200 MHz) the same as in Example 1; mp. 204°-207°C

### Example 3 - Preparation of 1-benzyl-4-(5,6-dimethoxy-1-oxo-indane-2-carbonyl)-pyridinium chloride (Formula XII)

5.0g (0.017mol) of 5,6-dimethoxy-2-(pyridine-4-carbonyl)indan-1-one was suspended in 40ml of cyclohexanol. The yellow suspension was stirred and heated to 125°C. After dissolving of the starting material, 9.75ml (0.085mol) of benzyl chloride was added into the suspension. Within 2 hours an orange product was formed. Thereafter, the reaction mixture was cooled to 110°C and then 20ml of toluene was added. The suspension was stirred at 90°C for 15 minutes and was then allowed to cool down to room temperature. The thick precipitate was filtered off and the product was washed with 10ml of acetone. The product was dried at 50°C, to give 5.5g of the title compound (yield: about 77%).
¹H NMR (CDCl₃, 400 MHz): 9.72 (d, 2), 8.48 (d, 2), 7.61-7.65 (m, 2), 7.43-7.48 (m, 3), 7.29 (s, 1), 7.04 (s, 1), 6.26 (s, 1), 4.04 (s, 3), 4.01 (s, 2), 3.96 (s, 3), 2.17 (s, 1); mp. 231-234°C.

### Example 4 - Preparation of 1-benzyl-4-(5,6-dimethoxy-1-oxo-indane-2-carbonyl)-pyridinium chloride (Formula XII)

5.0g (0.017mol) of 5,6-dimethoxy-2-(pyridine-4-carbonyl)indan-1-one was suspended in 50ml of cyclohexanol and 50ml of toluene. The yellow suspension was stirred and heated to 114°C. Then 9.75ml (0.085mol) of benzyl chloride was added into the suspension and stirred at reflux for 2 hours. An orange product was formed. After addition of toluene (20ml), the mixture was stirred for 10min at reflux and was then allowed to cool down to room temperature. The precipitate was filtered off and washed with 10ml of acetone. The product was dried at 50°C, to give 6.54g (yield: about 92%).
¹H NMR (CDCl₃, 400 MHz) the same as in Example 3; mp. 232°-234°C.

### Example 5 - Preparation of 1-benzyl-4-(5,6-dimethoxy-1-oxo-indane-2-carbonyl)-pyridinium chloride (Formula XII)

5.0g (0.017mol) of 5,6-dimethoxy-2-(pyridine-4-carbonyl)indan-1-one was suspended in 40ml of butanol and 40ml of toluene. The yellow suspension was stirred and heated up to 105°C. Then 9.75ml (0.085mol) of benzyl chloride was added into the suspension and stirred at reflux for 3 hours. An orange product was formed. After addition of toluene (20ml), the mixture was stirred for 10min at reflux and was then allowed to cool down to room temperature. The precipitate was filtered off and washed with 10ml of acetone. The product was dried at 50°C, to give 6.71g (yield: about 94%).
¹H NMR (CDCl₃, 400 MHz) the same as in Example 3; mp. 231°-233°C.

### Example 8 - Preparation of 1-benzyl-4-(5,6-dimethoxy-1-oxo-indane-2-carbonyl)-pyridinium chloride (Formula XII)

5.0g (0.017mol) of 5,6-dimethoxy-2-(pyridine-4-carbonyl)indan-1-one was suspended in 240ml of benzyl chloride. The yellow suspension was stirred and heated up till 177°C. The reaction was kept at boiling during 1h. The orange product was formed. After cooling of the mixture to 60°C, 20ml of acetone was added. The content of reaction was maintained at boiling for 10min and cooled down to room temperature. The precipitate was filtered off and poured into 20ml of acetone once again. The product was dried at 50°C, to give 7.04g (yield: about 98%).
¹H NMR (CDCl₃, 400 MHz) the same as in Example 3; mp. 231°÷232°C.

### Example 7 - Preparation of 1-benzyl-4-(5,6-dimethoxy-1-oxo-indane-2-carbonyl)-pyridinium bromide (Formula XII)

5.0g (0.017mol) of 5,6-dimethoxy-2-(pyridine-4-carbonyl)indan-1-one was suspended in 40ml of cyclohexanol and 40ml of toluene. The yellow suspension was stirred and heated to 114°C. Then 3ml (0.025mol) of benzyl bromide was added into the suspension and stirred at reflux for 3 hours. An orange product was formed. After addition of toluene (20ml), the mixture was stirred for 10min at reflux and next was allowed to cool to room temperature. The precipitate was filtered off and washed with 10ml of acetone. The product was dried at 50°C, to give 7.75g (yield: about 98%). Melting point: 234-237°C.

### Example 8 - Preparation of 1-benzyl-4-(5,6-dimethoxy-1-oxo-indane-2-carbonyl)-pyridinium bromide (Formula XII)

5.0g (0.017mol) of 5,6-dimethoxy-2-(pyridine-4-carbonyl)indan-1-one was suspended in 30cm³ of toluene and 30cm³ of butanol. The yellow suspension was stirred and heated up to reflux (103-105°C). Then 3cm³ of benzyl bromide was dropped into the suspension and further stirred at reflux for 4 hours. An orange product was plentifully formed. The reaction mixture was cooled down to about 20-25°C and crude product, which contains 5-7% of diketone, was filtered off and washed with 15cm³ of acetone. Still wet product was added into a reactor and treated with 37.5cm³ of dichloromethane. The suspension was well stirred for 10minutes and next 0.2cm³ of triethylamine diluted in 12.5cm³ of dichloromethane was added dropwise. The reaction mixture was stirred for 30minutes at temperature 20-25°C and all solid was filtered off and washed with 6.5cm³ of dichloromethane. There was obtained about 7.0g of dried product (yield: 89%). Melting point: 232-236°C.

### Example 9. - Preparation of 2-((1-benzyl-piperidin-4-yl)-hydroxy-methyl)-5,6-dimethoxy-indan-1-one) (Formula XIII)

A suspension of 2g 1-benzyl-4-(5,6-dimethoxy-1-oxo-indane-2-carbonyl)-pyridinium chloride (Formula XII) and 1g catalyst Pt/C in methanol, was hydrogenated under pressure of 5-6 bar at room temperature until the hydrogenation reaction was substantially complete. The progress of the reaction was monitored by the HPLC-MS method A. After the catalyst was filtered off, 5% sodium hydrogen carbonate aqueous solution was added, followed by the extraction with dichloromethane. The combined organic fractions were dried with anhydrous sodium sulfate. Sodium sulfate was filtered off, filtrate was concentrated under reduced pressure to afford crude compound XIII, which, after washing in methanol, gave white crystals of 2-((1-benzyl-piperidin-4-yl)-hydroxy-methyl)-5,6-dimethoxy-indan-1-one), (compound Formula XIII)
Mass spectrum m/e: 396 (M+H⁺); ¹H NMR (CDCl₃ 400 MHz) 1.22-1.36 (m, 2H), 1.56-1.63 (d, 1H), 1.77-2.07 (m, 4H), 2.80-2.92 (m, 3H), 2.92-3.00 (dd, 1H), 3.10-3.18 (m, 1H), 3.47 (s, 2H), 3.54-3.57 (m, 1H), 3.82 (s, 3H), 3.90 (s, 3H), 4.52 (d, 1H), 7.05 (s, 1H), 7.10 (s, 1H), 7.25-7.26 (m, 1H), 7.30-7.34 (m, 4H)

### Example 10 - Preparation of 2-[(1-benzyl-1,2,3,6-tetrahydro-pyridin-4-yl)-hydroxymethyl]-5,6-dimethoxy-indan-1-one (FormulaXIII)

To the orange suspension of 1-benzyl-4-(5,6-dimethoxy-1-oxo-indane-2-carbonyl)-pyridinium bromide (20g, 0.043mol) in 510cm³ of methanol, cooled down to about 10°C, was continuously dropped within 30minut a solution of sodium borohydride (4.04g, 0.107mol) and KOH (0.8g, 0.014mol) in 90cm³ of water. The internal temperature was kept in the range of 10-18°C. The reaction mixture was stirred for next 10min and 200ml of water was added followed by acidification with 8% hydrochloric acid to bring pH to -4, keeping the temperature below 20°C. Methanol was removed under reduced pressure at temp.50°C and obtained water liquor was cooled down to 20°C and treated with 60cm³ of dichloromethane and 10% KOH till pH was brought to -8, keeping temperature below 20°C. The products were extracted with all together 240cm³ of dichloromethane (4x60cm³). Collected organic layers were evaporated under reduced pressure to get an oily residue, which was dissolved in 40cm³ of hot *iso*-propanol. The solution was cooled down to room temperature and next was kept for 5 hours at 8 °C. The white crystals were filtered off, washed with cooled iso-propanol and dried to obtained mixture of products (11g).
This mixture was purified on flash column chromatography using gradient dichloromethane/methanol as eluent to get samples of two pure products with the same mass 393 (EI-MS) but different ¹H NMR results.
First compound ¹H NMR (DMSO, δ(ppm)): 1.82 (m, 2H), 2.36 (t, 2H), 2.73-3.05 (m, 5H), 3.42 (s, 2H), 3.77 (s, 3H), 3.84 (s, 3H), 4.27 (m, 1H), 4.99 (d, 1H), 5.55 (m, 1H), 7.02 (s, 1H), 7.10 (s, 1H), 7.18-7.32 (m, 5H).
Second compound ¹H NMR (DMSO, δ(ppm)): 1.96 (m, 1H), 2.92 (m, 1H), 2.41-2.58 (m, 2H), 2.74-2.97 (m, 5H), 3.52 (s, 2H), 3.76 (s, 3H), 3.83 (s, 3H), 4.43 (m, 1H), 4.81 (d, 1H), 5.64 (m, 1H), 7.02 (s, 1H), 7.08 (s, 1H), 7.21-7.34 (m, 5H).

### Example 11 - Preparation of 2-[(1-benzyl-1,2,3,6-tetrahydro-pyridin-4-yl)-hydroxymethyl]-5,6-dimethoxy-indan-1-one (FormulaXIII)

Into a reactor (250ml) 0.90g KOH (1.5eq) and 30cm³ of methanol is added, the mixture is stirred till the whole solid is dissolved. Then 5g of 1-benzyl-4-(5,6-dimethoxy-1-oxo-indane-2-carbonyl)-pyridinium (Formula XII) is added into the solution. After it is dissolved, a red product is precipitated right away. The stirring was continued at room temperature for 1hour and the solid was filtered off on a Schott funnel and washed with 15cm³ of methanol. The product was dried at room temperature to constant mass. Produced is about 4.5-5g of the potassium salt of 1-benzyl-4-[(5,6-dimethoxy-1-oxo-indan-2-ylidene)-hydroxy-methyl]-pyridinium ¹H NMR (DMSO, δ(ppm)): 3.43 (s, 2H), 3.70 (s, 3H), 3.78 (s, 3H), 5.79 (s, 2H), 6.87 (s, 1H), 7.00 (s, 1H), 7.44÷7.54 (m, 5H), 8.09 (d, 2H), 8.99 (d, 2H).

5g of the mentioned product is suspended in 200ml of methanol. The content of reactor is stirred well and cooled down using water bath to get +10°C. After 15minutes 1g of sodium borohydride (2.5eq calculated on 1-benzyl-4-(5,6-dimethoxy-1-oxo-indane-2-carbonyl)-pyridinium) is added in five portions within 10minutes keeping internal temperature in range of 10-19°C. After addition, reaction mixture is stirred and maintained at room temperature for 10 minutes. Then the light yellow solution is acidified with hydrogen chloride (as ethanolic solution) to pH ∼4 and left for one hour at room temperature. After this time the precipitate was filtered off and filtrate concentrated under vacuum to get a dark residue. Crystallization from ethanol gave mixture of products with mass 393 (EI-MS).

### Example 12 - Preparation of 2-[(1-benzyl-1,2,3,6-tetrahydro-pyridin-4-yl)-hydroxymethyl]-5,6-dimethoxy-indan-1-one (FormulaXIII)

To a solution of 3.58g (O.Olmol) of KOH in 700ml of methanol, cooled down to about 10°C, 30g (mol) of benzyldiketone (Formula XII) and 120ml of DMF was added. After 15 minutes of mixing a solution of sodium borohydride in 300ml of methanol was slowly dripped within 1 hour to form a red suspension. Temperature was kept in range: 15-20°C. After that time the reaction mixture was acidified with hydrochloric acid to pH ∼6 and methanol was distilled off under reduced pressure. To the yellow residue 200ml of water was added and product was extracted with methylene chloride (3x50ml). Collected organic layers were evaporated under reduced pressure to get residue. Crystallization from ethanol gave mixture of products with mass 393 (EI-MS).

### Example 13 - Preparation of 2-[(1-benzyl-1,2,3,6-tetrahydro-pyridin-4-yl)-hydroxymethyl]-5,6-dimethoxy-indan-1-one (FormulaXIII)

To the solution of 0.90g (0.016mol) of KOH in 150ml of methanol, cooled down to about 100C, 5g (0.01mol) of benzyldiketone (XII) was added. The suspension was stirred for 15min and then 1g (0.03mol) of sodium borohydride was batchwise added into reaction mixture within 10min. The temperature during reduction was kept in range of 10-190°C. The reaction mixture was stirred and maintained at room temperature for 15 minutes. After that time, reaction mixture was acidified with hydrochloric acid to pH∼4 and methanol was distilled off under reduced pressure. 90ml of ethanol was added to the residue and the suspension was stirred for 1h at boiling. Then the content of reactor was allowed to cool to room temperature. The thick slurry was next filtered off and the product was washed with 15ml of ethanol. The solid was suspended in 20ml of water and alkalized with 10% sodium hydroxide in water to pH∼8. The product of reduction was extracted from water phase with methylene chloride (3x20ml). Collected organic layers were evaporated under reduced pressure to get residue. Crystallization from *iso*-propanol gave mixture of products with mass 393 (EI-MS).

### Example 14 - Preparation of 2-[(1-benzyl-1,2,3,6-tetrahydro-pyridin-4-yl)-hydroxymethyl]-5,6-dimethoxy-indan-1-one (FormulaXIII)

To the solution of 0.28g (0.005mol) of KOH in 150ml of methanol, cooled down to about 10°C, 2g (0.004mol) of benzyldiketone (XII) was added. The suspension was stirred for 15min and then 0.40g (O.Olmol) of sodium borohydride was portionwise added into reaction mixture within 10min. The temperature during reduction was kept in range of 10-16°C. The reaction mixture was stirred and maintained at room temperature for 15 minutes. After that time reaction mixture was acidified with hydrochloric acid to pH ∼4 and methanol was distilled off under reduced pressure to get residue. Crystallization from *iso*-propanol gave mixture of products with mass 393 (EI-MS).

### Example 15 - Preparation of 2-[(1-benzyl-1,2,3,6-tetrahydro-pyridin-4-yl)-hydroxymethyl]-5,6-dimethoxy-indan-1-one (FormulaXIII)

To the orange suspension of 30g (0.06mol) of benzyldiketone (Formula XII) in 900ml of methanol, cooled down to about 10°C, 120ml of DMF was added. After 15 minutes of mixing, solution of 8.48g (0.22mol) of sodium borohydride in 300ml of methanol was dropped within 4 h. Temperature was kept in range: 15-20°C. Reaction mixture was acidified with hydrochloric acid to pH ∼6 and methanol was distilled off under reduced pressure. To a yellow residue 200ml of distilled water was added and product was extracted from water phase with methylene chloride (3x50ml). Collected organic layers were evaporated under reduced pressure to get residue. Crystallization from *iso*-propanol gave mixture of products with mass 393 (EI-MS).

### Example 16 - Preparation of 2-[(1-benzyl-1,2,3,6-tetrahydro-pyridin-4-yl)-hydroxymethyl]-5,6-dimethoxy-indan-1-one (FormulaXIII)

To the orange suspension of 5g (0.01mol) of benzyldiketone (Formula XII) in 200ml of methanol, cooled down to about 10°C, solution of 1.00g (0.04 mol) of sodium borohydride in 50ml of water with addition of 0.55 g of KOH was slowly dropped (about 20 minutes). Temperature was kept in range: 15-20°C. After that time reaction mixture was acidified with hydrochloric acid to pH ∼6 and methanol was distilled off under reduced pressure. The product of reduction was extracted from a white residue suspended water phase with methylene chloride (3x10ml). Collected organic layers were evaporated under vacuum to get residue. Crystallization from *iso*-propanol gave mixture of products with mass 393 (EI-MS).

### Example 17 - Preparation of donepezil (Formula XV)

2-((1-benzyl-piperidin-4-yl)-hydroxy-methyl)-5,6-dimethoxy-indan-1-one) (from Example 9) was dissolved in dichloromethane and conc. sulfuric acid was added. The resulting reaction mixture was stirred at 35°C for 4-5 hours. The progress of the reaction was monitored by the HPLC-MS method A. The mixture was poured on ice water and the solution was extracted with dichloromethane. The combined organic fractions were washed with deionized water, dried with anhydrous sodium sulfate and concentrated under reduced pressure. In the concentrated solution the catalyst Pt/C and methanol were added. The suspension was hydrogenated under the pressure of 5-6 bar at the room temperature till the hydrogenation reaction was substantially complete. The catalyst was filtered off, 5% sodium hydrogen carbonate aqueous solution was added and the mixture was extracted with dichloromethane. The combined organic fractions were dried with anhydrous sodium sulfate and solvent was removed by the evaporation. The black oil residue was purified by the column chromatography (silicagel; Toluene : acetone : ammonia 25% = 30 : 14 : 0.5) yielding the crude donepezil, purity 32.7%, by the HPLC-MS method A, Rt -8.7 min.
Mass spectrum m/e: 380 (M+H⁺).

### Example 18 - Preparation of donepezil hydrochloride (Formula XV)

1,2g of 2-[(1-benzyl-1,2,3,6-tetrahydro-pyridin-4-yl)-hydroxy-methyl]-5,6-dimethoxy-indan-1-one (both compounds from Example 10) were dissolved in 8ml iso-propanol and next acidified with 0.85 ml ∼24% HCl/ EtOH diluted in 3.5ml iso-propanol. The mixture was stirred under reflux for 1h and was allowed to cool down to room temperature. The obtained thick slurry was next filtered off and the product was washed with 2x3ml of iso-propanol and 5ml of acetone, dried at 60°C to constant mass. (EI MS: 375). ¹H NMR (DMSO, δ(ppm)): 2,89 (br s, 2H), 3,20 (m, 1H), 3,56 (br s, 1H), 4,00-3,74 (m, 4H), 3,82 (s, 3H), 3,90 (s, 3H), 4,40 (d, 2H), 6,30 (br s, 1H), 7,00 (s, 1H), 7,15 (s, 1H), 7,20 (s, 1H), 7,48 (br s, 3H), 7,63 (br s, 2H).

A suspension of 1g of obtained compound and 60mg of Pt-catalyst in a mixture of methanol and dichloromethane, was hydrogenated under a pressure of 5-6 bar at room temperature until the hydrogenation reaction was substantially complete. The progress of the reaction was monitored by the HPLC-MS-UV method A. After the catalyst was filtered off, the filtrate was concentrated under reduced pressure. The resulting oil residue was dissolved in absolute ethanol and hydrogen chloride (in ethanol solution) was added. Diisopropyl ether was added to the solution to obtain crude compound of donepezil hydrochloride.
Mass spectrum m/e: 380 (M+H⁺).

### The method A:

A suitable analytical HPLC instrument with UV and MS detector (e.g. Agilent 1100)
Column: YMC-Pack Pro C18, 150x4,6 mm ID, S-3 µm, 12 nm
Column temperature: 35°C
Flow rate: 1 ml/min
Detector:
   a) DAD: wavelength: 240 nm, Bw 8 nm; Ref. Signal 360 nm, Bw 100 nm
   b) MSD / Ion Trap: ES ionization; Capillary 3500 V, Nebuliser 40.0 psi, Dry Gas 10.01/min, Dry Temp. 350 °C, Skimmer 40 V, Cap. Exit 166 V, Target 30000, Max. Accu. Time 300.00 ms
Injection volume: 3 µl
Mobile phase: A: B = 90% v/v : 10% v/v
   A) 40 mM NH₄OAc (pH=5.0)
   B) CH₃CN
Elution: Gradient program: 0 min→10% B, 15 min→90% B, 15.10 min→90% B,
   20 min→10% B
Stop time: 20 min

### Example (19) - Preparation of donepezil hydrochloride (Formula XV)

2 g (0.004 mol) of 1-benzyl-4-(5,6-dimethoxy-1-oxo-indane-2-carbonyl)-pyridinium bromide (Formula XII) was suspended in 100 ml of methanol, then 0.505 g of the Pt/C catalyst and 356 µl of concentrated sulphuric acid (0.006 mol) were added into the suspension. The suspension was hydrogenated under pressure of 10 bars and temperature of 35°C until the hydrogenation reaction was completed. The progress of the reaction was monitored by the HPLC-MS method A (described in Example 18). After the catalyst was filtered off, the filtrate was concentrated under reduced pressure. The resulting oil residue was dissolved in dichloromethane and pH value was adjusted to 6-7 with 1 % aqueous solution of NaOH. The organic layer was separated, washed with water and dried with anhydrous sodium sulphate. Sodium sulphate was filtered off and filtrate was concentrated under reduced pressure to afford oil residue of donepezil. The oil residue was dissolved in methanol. The solution was cooled down at 0-5 °C, and then solution of concentrated hydrochloride in methanol (0.54 g conc. HCl/1.5 ml methanol) was added. *tert*-Buthylmethyl ether was added into the solution to obtain crude compound of donepezil hydrochloride.
Mass spectrum m/e: 380 (M+H⁺).

### Example 20 Preparation of donepezil hydrochloride hemihydrate

### Example I

15,0 g of donepezil hydrochloride was suspended in 75 ml of methanol and 0,5 ml of deionised water. The suspension was then heated at 55°C until all crystals were dissolved. The solution was filtered and cooled down at 0-5°C. 100 ml of tert-butyl methyl ether was added drop wise in the cooled stirred solution during 40 minutes. Stirring was continued for 30 minutes at 0-5 °C. The precipitate was filtered, washed with tert-butyl methyl ether and dried at 40°C and 10 mbar during 1 hour to yield 13,2 g of donepezil hydrochloride hemihydrate (water content 2,67 %weight).

### Example II

12,5 g of donepezil hydrochloride was suspended in 37,5 ml of methanol and 0,4 ml of deionised water. The suspension was then heated at 60°C, until all crystals were dissolved. The obtained solution was then filtered and cooled down at room temperature. 200 ml of ethyl acetate was charged in 250 ml reactor vessel and cooled down at 0°C. In the stirred, cooled ethyl acetate, filtered donepezil hydrochloride solution was added. The solution was stirred for 30 minutes. Obtained precipitate was filtered, washed with ethyl acetate and dried at 40°C and 10 mbar during 1 hour to yield 9,95 g of donepezil hydrochloride hemihydrate

### Example 21 Preparation of donepezil hydrochloride monohydrate

4,0 g of donepezil hydrochloride was suspended in 20 ml of methanol and 0,2 ml of deionised water. The suspension was then heated at 40°C, until all crystals were dissolved. The obtained solution was then filtered, cooled down at 0-5°C and stirred for 10 minutes (during that time crystallization started). Then 24 ml of tert-butyl methyl ether was added drop wise into the suspension during 20 minutes. Stirring was continued for 15 minutes at 0-5 °C. The precipitate was filtered, washed with tert-butyl methyl ether and dried at 40°C and 10 mbar during 1 hour to yield 3,12 g of donepezil hydrochloride form I (water content 4,13 %weight).

### Example 22 Preparation of donepezil hydrochloride sesquihydrate

4,0 g of donepezil hydrochloride was suspended in 20 ml of methanol and 0,2 ml of deionised water. The suspension was then heated at 40°C, until all crystals were dissolved. The obtained solution was then filtered, cooled down at 0-5°C and stirred for 10 minutes (during that time crystallization started). Then 24 ml of tert-butyl methyl ether was added drop wise into the suspension during 20 minutes. Stirring was continued for 15 minutes at 0-5 °C. The precipitate was filtered, washed with tert-butyl methyl ether and dried at 40°C and 10 mbar during 1 hour. The crystal was then put over water on room temperature and after 24 h donepezil hydrochloride sesquihydrate was isolated.

### Example 23 Preparation of donepezil hydrochloride dihydrate

4,0 g of donepezil hydrochloride was suspended in 20 ml of methanol and 0,2 ml of deionised water. The suspension was then heated at 40°C, until all crystals were dissolved. The obtained solution was then filtered, cooled down at 0-5°C and stirred for 10 minutes (during that time crystallization started). Then 24 ml of tert-butyl methyl ether was added drop wise into the suspension during 20 minutes. Stirring was continued for 15 minutes at 0-5 °C. The precipitate was filtered, washed with tert-butyl methyl ether and dried at 40°C and 10 mbar during 1 hour. The crystal was then put over water on 40°C and after 10 days donepezil hydrochloride dihydrate was isolated.

## Claims

1. A process for the preparation of donepezil, or a pharmaceutically-acceptable salt thereof, which process comprises;
A) reducing the compound of Formula XII 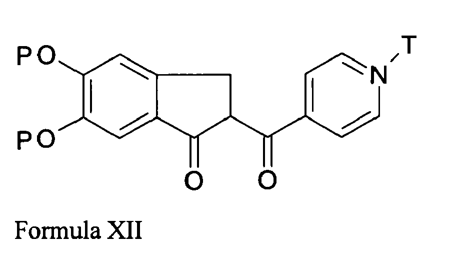 wherein P is a precursor group capable of conversion to a methyl group, alternatively P is a methyl group, wherein T is a precursor group capable of conversion to a benzyl group or T is a hydrogen atom, alkyl or an aralkyl,
to obtain compound of Formula XIII 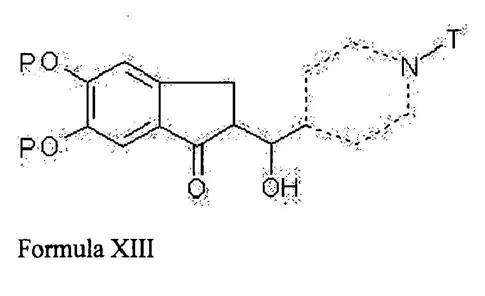 wherein the dotted lines represent a bond that may exist as double or as a single bond,
wherein P is a precursor group capable of conversion to a methyl group, alternatively P is a methyl group and wherein T is a precursor group capable of conversion to a benzyl group or T is a hydrogen atom, alkyl or an aralkyl;
B) dehydrating and subsequently reducing a compound of Formula XIII where P is a methyl group and T is a benzyl group to obtain a compound of Formula I (donepezil, or a pharmaceutically-acceptable salt thereof), or additionally
C) where P is a methyl group and T is not a benzyl group, converting the group T to a benzyl group to obtain donepezil, or a pharmaceutically-acceptable salt thereof, or
D) where P is a precursor group capable of conversion to a methyl group and T is a benzyl group, then converting P to a methyl group to obtain donepezil, or a pharmaceutically-acceptable salt thereof, or
E) where P is a precursor group capable of conversion to a methyl group and T is not a benzyl group, undertaking steps C) and D), in either order, to obtain donepezil, or a pharmaceutically-acceptable salt thereof.

2. Process as claimed in claim 1, wherein the process additionally comprises;
A) direct reduction of the compound of Formula XII where P is methyl group and T is benzyl group 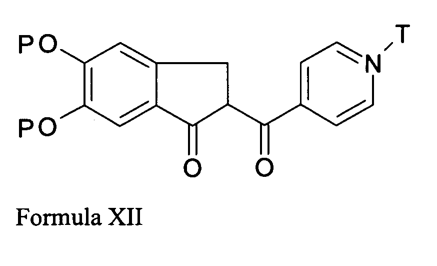 to obtain a compound of Formula I (donepezil, or a pharmaceutically-acceptable salt thereof) without isolating Formula XIII as an intermediate; or additionally
B) where P is a methyl group and T is a precursor group capable of conversion to a benzyl group, converting the group T to a benzyl group to obtain donepezil, or a pharmaceutically-acceptable salt thereof, or
C) where P is a precursor group capable of conversion to a methyl group and T is a benzyl group, then converting P to a methyl group to obtain donepezil, or a pharmaceutically-acceptable salt thereof, or
D) where P is a precursor group capable of conversion to a methyl group and T is a precursor group capable of conversion to a benzyl group, undertaking the above described steps B) and C) of converting P to a methyl group and converting group T to a benzyl group in either order, to obtain donepezil, or a pharmaceutically-acceptable salt thereof

3. A process as claimed in claim 1, wherein the process additionally comprises an initial step of converting a compound of Formula IX 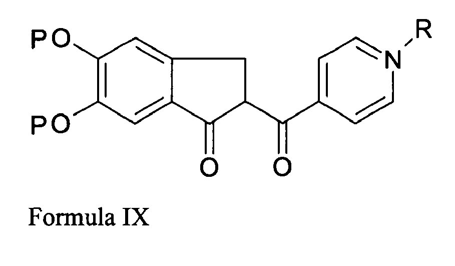 wherein P is a precursor group capable of conversion to a methyl group, alternatively P is a methyl group, and R is a hydrogen atom, alkyl or an aralkyl, to obtain a compound of Formula XII (wherein the group R is not the same as the group T).

4. A process for the preparation of donepezil, or a pharmaceutically-acceptable salt thereof, utilizing, as an intermediate in the process, a compound of Formula IX 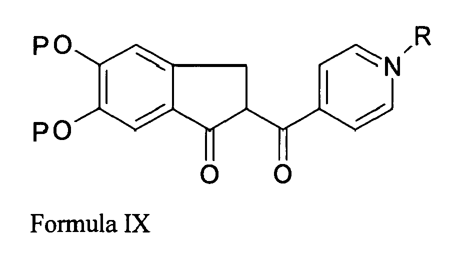 wherein P is a precursor group capable of conversion to a methyl group, alternatively P is a methyl group, and R is a hydrogen atom, alkyl or an aralkyl, in either of its tautomeric forms or in the form of any salt thereof.

5. A process for the preparation of donepezil, or a pharmaceutically-acceptable salt thereof, utilizing as an intermediate in the process a compound of Formula XIII 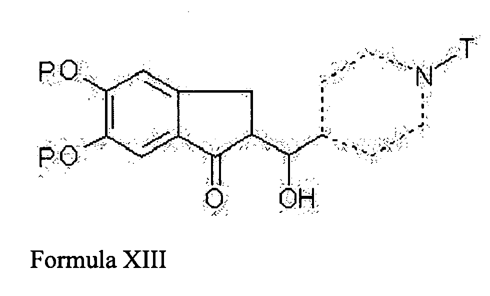 wherein the dotted lines represent a bond that may exist as double or as a single bond [preferably a only one bond is a double bond in the ring alternatively all of the bonds in the ring are saturated], wherein P is a precursor group capable of conversion to a methyl group, alternatively P is a methyl group and wherein T is a precursor group capable of conversion to a benzyl group or T is a hydrogen atom, alkyl or an aralkyl

6. A compound of Formula IX, as defined in claim 4, wherein in addition the carbonyl group on the indanone group may be optionally protected.

7. A compound of Formula XIII, as defined in claim 5, wherein in addition the carbonyl group on an indanone group may be optionally protected.

8. A process for the preparation of a compound of Formula IX comprising;
reacting a compound of Formula X 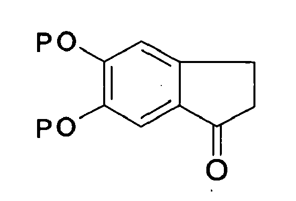 Formula X
wherein P is as defined in claim 1 or 5, with a compound of Formula XI 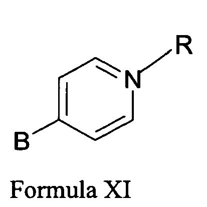 wherein R is a precursor group capable of conversion to a benzyl group or R is a hydrogen atom, alkyl or an aralkyl and B represents the carboxylic acid group COOH or any of its derivatives such as esters, halides, anhydrides or salts of any thereof, to form a compound of Formula IX.

9. A process as claimed in claim 8, wherein the process is conducted in the presence of alkali metal alkoxide, such as sodium methoxide, or an alkali metal hydride.

10. A process as claimed in claim 8 or 9, wherein the reaction is conducted in the presence of aliphatic alcohols as an initiator.

11. A process as claimed in any claim from 8 to 10, wherein the reaction is conducted at an elevated temperature in the range of from 100°C to 180°C.

12. A process as claimed in claim from 8 to 11, wherein the reaction is carried out at reflux.

13. A process as claimed in any claim from 8 to 12, wherein the reaction is conducted in the presence of solvent or without solvent.

14. A process as claimed in claim 13, wherein the reaction is conducted in an aromatic solvent.

15. A process as claimed in claim 14, wherein the solvent is selected from cyclohexanol, butanol, toluene or a mixture thereof.

16. A process as claimed in claim 1, wherein step A) of claim 1 is conducted in the presence of a hydride.

17. A process as claimed in claim 1, wherein step B) of claim 1 is conducted with employment of hydrogen or a source of hydrogen.

18. A process as claimed in claim 17, wherein the hydrogenation is conducted with the presence of a metal catalyst.

19. A process as claimed in claim 2, wherein step A) of claim 2 is conducted with employment of hydrogen or a source of hydrogen.

20. A process as claimed in claim 19, wherein the hydrogenation is conducted with the presence of a metal catalyst,.
